# EUROPEAN PATENT SPECIFICATION

(11) **EP 3 292 787 B1**
(45) Date of publication and mention of the grant of the patent: **23.12.2020**
(21) Application number: 17184321.2
(22) Date of filing: 01.08.2017
(51) Int. Cl.: A45D 34/00, A45D 40/00, A61Q 1/00, A61Q 5/00, A61Q 11/00, A61Q 17/00, A61J 1/00, A61K 8/06, A61K 9/10, A61Q 19/00, A61K 8/03, B65D 77/00, B65D 77/08, B65D 35/22, A61K 9/107, A61K 9/06, A45D 40/24, A61K 9/00

(54) **PACKAGING SYSTEM FOR DEPENDENCY-FREE USE OF PRODUCTS FOR TOPICAL APPLICATION**
VERPACKUNGSSYSTEM ZUR ABHÄNGIGKEITSFREIEN VERWENDUNG VON PRODUKTEN ZUR TOPISCHEN ANWENDUNG
SYSTÈME D'EMBALLAGE POUR UTILISATION SANS DÉPENDANCE DE PRODUITS POUR APPLICATION TOPIQUE

(30) Priority: 08.09.2016 IT 201600090778
(43) Date of publication of application: 14.03.2018
(73) Proprietor: ITALIAN GROUP S.r.l., 25124 Brescia (IT)
(72) Inventor: MORA, Valtiero, I-25124 BRESCIA (IT)
(74) Representative: Gualeni, Nadia

(56) References cited:
- JP-A- S59 127 646
- US-A- 4 778 052
- HUGHES ERIC ET AL: "Microfluidic preparation and self diffusion PFG-NMR analysis of monodisperse water-in-oil-in-water double emulsions.", JOURNAL OF COLLOID AND INTERFACE SCIENCE 01 JAN 2013, vol. 389, no. 1, 1 January 2013 (2013-01-01), pages 147-156, XP002768921, ISSN: 1095-7103
- Dolimite: "Double Emulsion System creates monodisperse double emulsions", , June 2014 (2014-06), XP002768922, Retrieved from the Internet: URL:https://www.manufacturingchemist.com/n ews/article_page/Double_Emulsion_System_cr eates_monodisperse_double_emulsions/99479 [retrieved on 2017-04-05]
- FRENKEL M ET AL: "Multiple emulsions - I. Stability: Inversion, apparent and weighted HLB", ANALYTICAL SCIENCES, THE JAPAN SOCIETY FOR ANALYTICAL CHEMISTRY, US, vol. 94, no. 1, 1 July 1983 (1983-07-01), pages 174-178, XP024187874, ISSN: 0021-9797, DOI: 10.1016/0021-9797(83)90247-3 [retrieved on 1983-07-01]

## Description

The present invention relates to a packaging system, in a same container, of products for topical application.

In the field of dermatology and cosmetics, it is known to use products containing active substances for topical applications. These products are commonly in the form of emulsions, creams, ointments, pastes, salves, gels, lipogels and the like.

In the field, the problem of loss of efficacy in the continued application of functional dermocosmetic products containing cosmetically active substances is known. After a significant initial improvement, despite the continued, constant and prolonged application of the product itself, the beneficial effects are significantly reduced or even cease altogether.

This phenomenon, well known in the pharmaco-biochemical field, is explained by the dependency or habit, namely by a particular form of adaptation of the organism that, in order to defend itself from the repeated and continuous administration of heterologous substances and biologically and pharmacologically active ingredients becomes increasingly less sensitive and less reactive to these substances.

Functional cosmetic products can be grouped into two categories:
a) products for continuous and daily use;
b) overactive impact products for periodic or cyclical use.

A known remedy against the phenomenon of dependency for continuous use products is the alternate use of different products which, although having the same cosmetic purpose (for example, protective, moisturizing, nourishing, normalizing), are formulated with completely different substances, possibly belonging to different domains, namely vegetable or animal derivatives.

An example of a known packaging system for products for topical application that allows overcoming the phenomenon of dependency is shown in patent document EP0243321, which describes a container that contains two or more layers of different products as a formulation and/or as a dosage of active substances. Another example is disclosed in US 4 778 052 A. The layered products are then dispensed and used consecutively to alternate the use thereof or to obtain progressively greater concentrations of active substances.

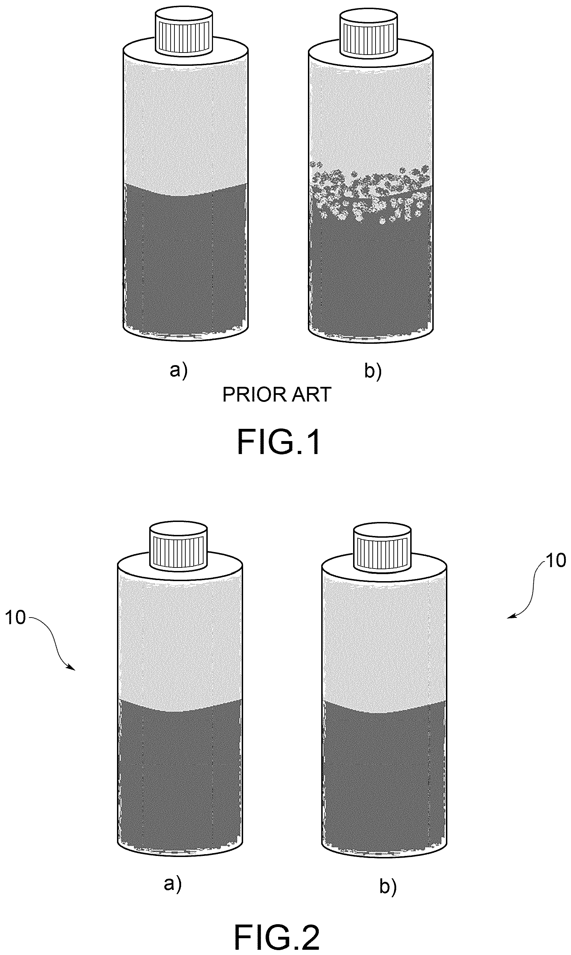

However, this known system has a considerable drawback, related to the fact that over time the active ingredients in the various products (as formulation and/or as a dosage of active substances) tend to move from one product to the other (as shown in figure 1b)). Figure 1 shows an example of a known container in which a layer of a first product is housed, containing a first active substance and a layer of a second product containing a second active substance and arranged in contact with the first product. In a newly filled container (figure 1a)), the products are divided in layers. Over time, the first active substance contained in the first product tends to move into the second product and/or the second active substance contained in the second product tends to move into the first product (figure 1b)), making the division in layers quite vain and the alternate use impossible.

The object of the present invention is to implement a packaging system that allows arranging layers of different products (as a formulation and/or as a dosage of active substances) in contact with each other and ensuring the separation thereof over time.

Such an object is achieved by a packaging system according to claim 1.

Advantageously, a packaging system according to the present invention allows for the alternate dispensing of the layered products over time.

The features and the advantages of the packaging system according to the present invention will appear more clearly from the following description, made by way of an indicative and non-limiting example with reference to the accompanying figures, in which:
- figure 1 shows a packaging system of the prior art, in a step immediately following the packaging (a) and after a certain period of time (b), in which the spread of the active substances between two different products contained in the container is clear;
- figure 2 shows a packaging system according to the invention, in a step immediately following the packaging (a) and after a certain period of time (b), in which there is no spread of the active substances between the two products contained in the container;

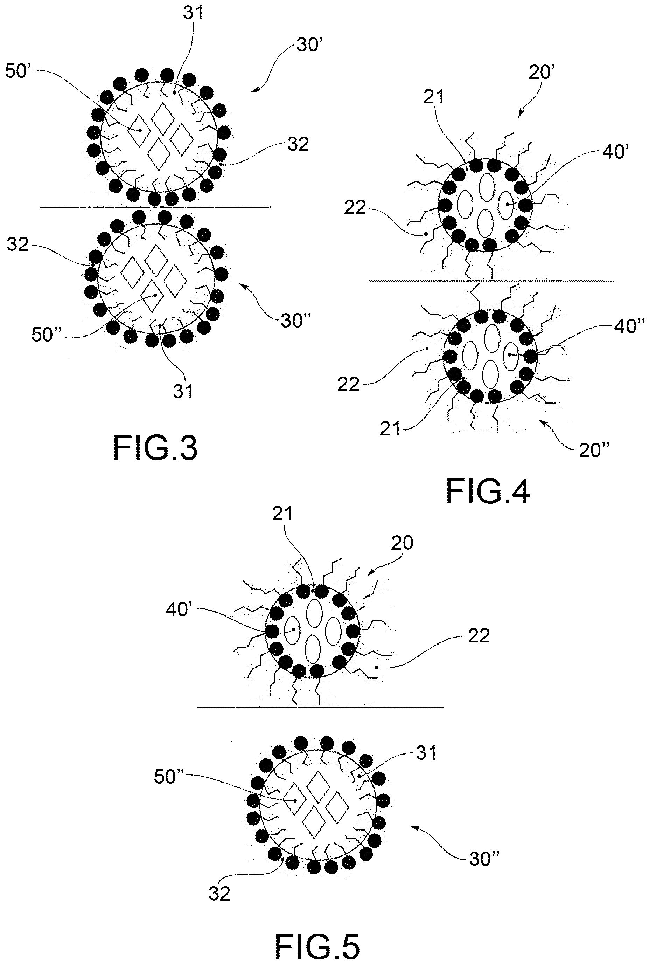

- figure 3 shows a first example of the packaging system according to the invention, in particular a layer of a first product (oil-in-water emulsion) containing a first active substance (fat-soluble), in contact with a layer of a second product (oil-in-water emulsion) containing a second active substance (fat-soluble);
- figure 4 shows a second example of the packaging system according to the invention, in particular a layer of a first product (water-in-oil emulsion) containing a first active substance (water soluble), in contact with a layer of a second product (water-in-oil emulsion) containing a second active substance (water soluble);
- figure 5 shows a third example of the packaging system according to the invention, in particular a layer of a first product (water-in-oil emulsion) containing a first active substance (water soluble), in contact with a layer of a second product (oil-in-water emulsion) containing a second active substance (fat-soluble);

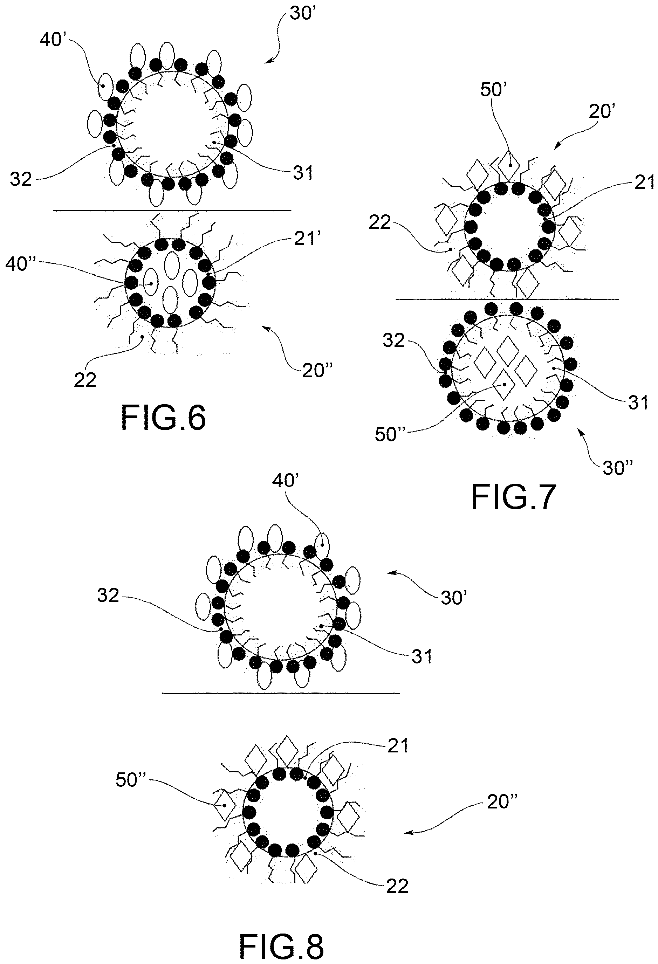

- figure 6 shows a fourth example of the packaging system according to the invention, in particular a layer of a first product (oil-in-water emulsion) containing a first active substance (water soluble), in contact with a layer of a second product (water-in-oil emulsion) containing a second active substance (water soluble);
- figure 7 shows a fifth example of the packaging system according to the invention, in particular a layer of a first product (water-in-oil emulsion) containing a first active substance (fat-soluble), in contact with a layer of a second product (oil-in-water emulsion) containing a second active substance (fat-soluble);
- figure 8 shows a sixth example of the packaging system according to the invention, in particular a layer of a first product (oil-in-water emulsion) containing a first active substance (water soluble), in contact with a layer of a second product (water-in-oil emulsion) containing a second active substance (fat-soluble);

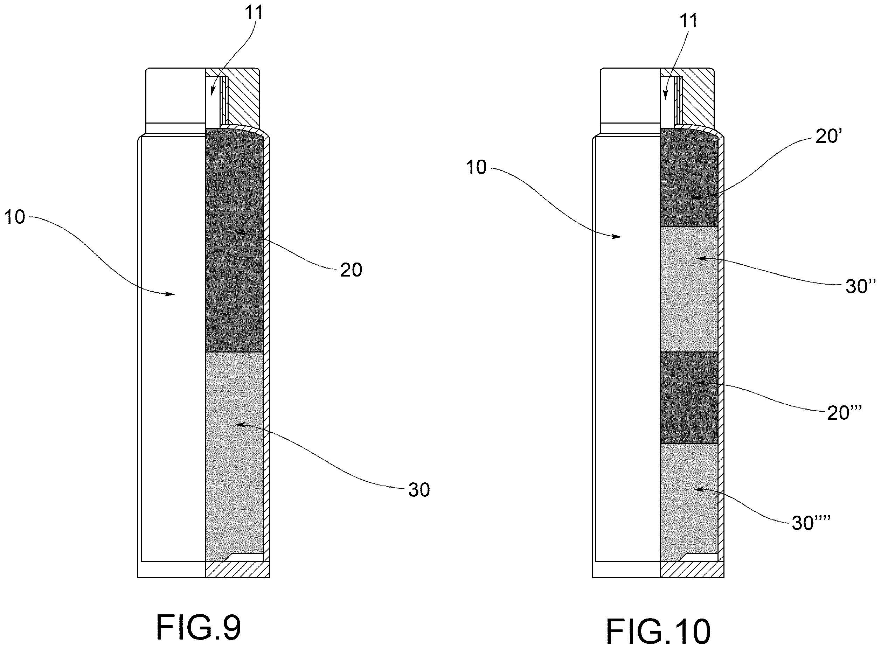

- figure 9 shows a container filled with the packaging system according to the invention, in which two layers of different products are contained;
- figure 10 shows a container filled with the packaging system according to the invention, in which more than two layers of different products are contained;

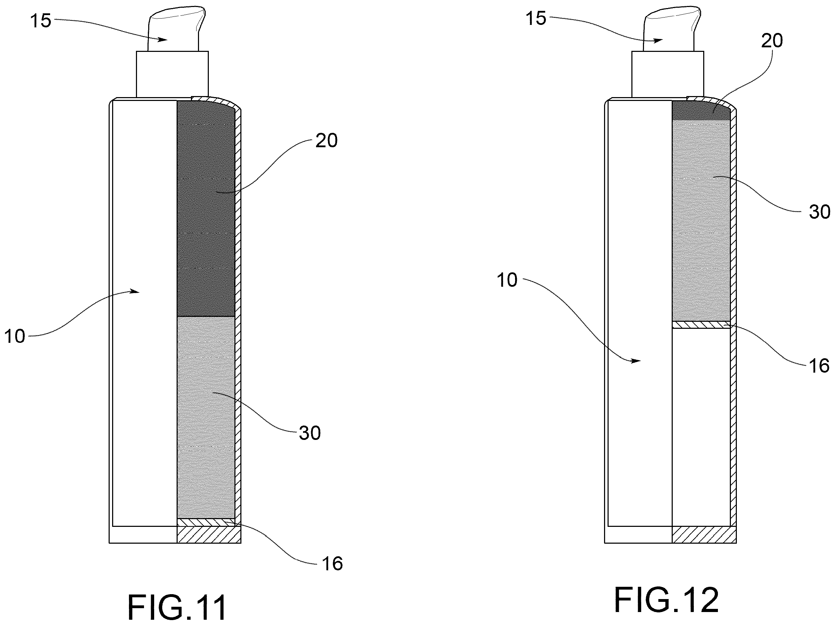

- figures 11 and 12 show an airless container filled with the packaging system according to the invention, in which two layers of different products are contained, in an initial and an intermediate usage step.

The object of the present invention is a packaging system that involves placing two or more products 20, 30 in a same container 10, in layers or overlapping phases, so as to be used separately and in succession.

These products are as diverse as formulation and/or as active ingredients (20, 30), or they are different as concentration of the same active substance (20', 20'', 20''', 20"") in order to prevent the onset of the dependency process.

These products are in cream form.

The cream is substantially an oil in water emulsion 30 (aqueous cream) or a water in oil emulsion 20 (oily cream).

Emulsions are mainly classified as:

O/W (oil in water) : in which the outer layer (or phase) is continuous aqueous, and the inner layer (or phase) is dispersed oily.

W/O (water in oil) : in which the outer layer (or phase) is continuous oily, and the inner layer (or phase) is dispersed aqueous.

Moreover, the oily phase is a phase consisting of oil and all that is fat-soluble; and the aqueous phase is a phase consisting of water and all that is water soluble.

Because of the prevalent constituent, creams may be divided into hydrophobic creams and hydrophilic creams.

Hydrophobic cream or oily cream or water in oil cream (indicated as "W/O cream" with reference numeral 20): includes an inner phase hydrophilic 21 (water) and an outer lipophilic phase 22 (oil).

Hydrophilic cream or aqueous cream or oil in water cream (indicated as "O/W cream" with reference numeral 30): includes an inner lipophilic phase 31 (oil) and an outer hydrophilic phase 32 (water).

Cream 20, 30 contains at least one active ingredient 40, 50. Preferably, at least one active ingredient 40, 50 is present in each cream 20, 30.

This active ingredient 40, 50 is contained in the inner phase 21, 31 or in the outer phase 22, 32 of the individual molecules composing the cream itself.

The active ingredient is water soluble 40 or fa-soluble 50.

In cream 20, 30 the water soluble active ingredient 40 is contained in the hydrophilic phase.

In cream 20, 30 the fat-soluble active ingredient 50 is contained in the lipophilic phase.

In one example, both creams 20, 30 contain a water soluble 40 (figures 4, 6) or fat-soluble 50 (figures 3, 7) active ingredient.

In a different example (figures 5, 8), a first cream 20', 30' contains a water-soluble active ingredient 40' and a second cream 20", 30" contains a fat-soluble active ingredient 50".

Once the two creams 20, 30 have been contacted, in order to prevent the active ingredients 40, 50 contained therein from spreading between the two creams, thus making the division into layers altogether vain, it is necessary to ensure that there is at least one separation layer between the active ingredients 40, 50 that is not able to absorb such an active ingredient.

Specifically, in the case of water soluble active ingredient 40, it is necessary to ensure that there is at least one hydrophobic separation layer. In the case of fat-soluble active ingredient 50, it is necessary to ensure that there is at least one lipophobic separation layer.

If the active ingredient 40, 50 is contained in the inner phase 21, 31 of the individual molecules that make up cream 20, 30 (figures 3 to 5), the active ingredients 40, 50 contained therein do not spread and do not move from one cream to the other because they are already enclosed by the outer layer 22, 33 of molecules in which they are contained, which per se acts as a separation layer not able to absorb the active ingredient. In this example, therefore, the separation layer is the outer layer 22, 33 of the molecules that make up cream 20', 30' itself in which the active ingredient 40, 50 is contained.

If one of the active ingredients 40, 50 is contained in the outer phase 22, 32 of the individual molecules that make up the cream itself (figures 6 and 7), such an active ingredient 40, 50 does not move from one cream to the other only if the outer layer 22, 32 of the adjacent molecule acts as a separation layer not able to absorb the active ingredient. In this example, therefore, the separation layer is the outer layer of the molecules that make up the adjacent cream 20", 30".

In particular, if the active ingredient is water soluble and 40 contained in the outer phase 32, the outer layer 22 of the adjacent molecule is a hydrophobic separation layer (figure 6), i.e. the separation layer is the hydrophobic outer layer 22 of a second cream 20".

If the active ingredient is fat-soluble and 50 contained in the outer phase 22, the outer layer 32 of the adjacent molecule is a lipophobic separation layer (figure 7), i.e. the separation layer is the lipophobic outer layer 32 of a second cream 30".

If the active ingredients 40, 50 are both contained in the outer phase 22, 32 of the individual molecules that make up the cream itself (figure 8), the active ingredients 40, 50 contained therein do not move from one cream to the other only if the outer layer 22, 32 of the adjacent molecule acts as a separation layer not able to absorb the active ingredient.

In particular, since the active ingredients are one water soluble 40 and one fat-soluble 50, the presence of both a hydrophobic outer separation layer 21 and a lipophobic outer separation layer 32 is required.

Figure 3 shows a first embodiment example of the packaging system according to the invention.

In this example, a layer of a first oil in water cream 30' containing a first fa-soluble active ingredient 50' is contacted with a layer of a second oil in water cream 30" containing a second fat-soluble active ingredient 50".

The first fat-soluble active ingredient 50' is different from the second fat-soluble active ingredient 50'', or the first fat-soluble active ingredient 50' is different in concentration from the second fat-soluble active ingredient 50".

Both active ingredients 50', 50" are contained in the lipophilic phase, i.e. in the inner phase 31 of the individual molecules composing cream 30', 30".

In order to ensure the separation over time between creams 30', 30", avoiding mixing the fat-soluble active ingredients 50', 50", among the fat-soluble active ingredients there is at least one lipophobic separation layer, represented by the outer phase 32 of the individual molecules that make up cream 30', 30".

In this example, among the fat-soluble active ingredients 50', 50" are two lipophobic separation layers represented by the outer phases 32 of the individual molecules that make up creams 30', 30".

Figure 4 shows a second embodiment example of the packaging system according to the invention.

In this example, a layer of a first water in oil cream 20' containing a first water soluble active ingredient 40' is contacted with a layer of a second water in oil cream 20" containing a second water soluble active ingredient 40".

The first water soluble active ingredient 40' is different from the second water soluble active ingredient 40", or the first water soluble active ingredient 40' is different in concentration from the second water soluble active ingredient 40".

Both active ingredients 40', 40" are contained in the hydrophilic phase, i.e. in the inner phase 21 of the individual molecules composing cream 20', 20".

In order to ensure the separation over time between creams 20', 20", avoiding mixing the water soluble active ingredients 40', 40", among the water soluble active ingredients there is at least one hydrophobic separation layer, represented by the outer phase 22 of the individual molecules that make up cream 20', 20".

In this example, among the water soluble active ingredients 40', 40" are two hydrophobic separation layers represented by the outer phases 22 of the individual molecules that make up creams 20', 20".

Figure 5 shows a third embodiment example of the packaging system according to the invention.

In this example, a layer of a first water in oil cream 20' containing a first water soluble active ingredient 40' is contacted with a layer of a second oil in water cream 30" containing a fat-soluble active ingredient 50".

The first 40' and the second 50" active ingredient are different.

The first water soluble active ingredient 40', is contained in the hydrophilic phase, i.e. in the inner phase 21 of the individual molecules composing the water in oil cream 20'. The second fat-soluble active ingredient 50', is contained in the lipophilic phase, i.e. in the inner phase 31 of the individual molecules composing the oil in water cream 30".

In order to ensure the separation over time between creams 20', 30", preventing the active ingredients from moving from one cream to the other, among the active ingredients there both a lipophobic separation layer and a hydrophobic separation layer.

In this example, therefore, between the active ingredients there is both a lipophobic separation layer, represented by the outer phase 22 of the individual molecules that make up the first water in oil cream 20', and a hydrophobic separation layer, represented by the outer phase 32 of the individual molecules that make up the first oil in water cream 30".

Figure 6 shows a fourth embodiment example of the packaging system according to the invention.

In this example, a layer of a first oil in water cream 30' containing a first water soluble active ingredient 40' is contacted with a layer of a second water in oil cream 20" containing a second water soluble active ingredient 40".

The first water soluble active ingredient 40' is different from the second water soluble active ingredient 40", or the first water soluble active ingredient 40' is different in concentration from the second water soluble active ingredient 40".

The first water soluble active ingredient 40', is contained in the hydrophilic phase, i.e. in the outer phase 32 of the individual molecules composing the oil in water cream 30'. The second water soluble active ingredient 40', is contained in the hydrophilic phase, i.e. in the inner phase 21 of the individual molecules composing the water in oil cream 20".

In order to ensure the separation over time between creams 30', 20", avoiding mixing the water soluble active ingredients 40', 40", among the water soluble active ingredients there is at least one hydrophobic separation layer.

In this example, among the water soluble active ingredients 40', 40" is only one hydrophobic separation layers, represented by the outer phase 22 of the individual molecules that make up the second water in oil cream 20".

Figure 7 shows a fifth embodiment example of the packaging system according to the invention.

In this example, a layer of a first water in oil cream 20' containing a first fat-soluble active ingredient 50' is contacted with a layer of a second oil in water cream 30" containing a fat-soluble active ingredient 50".

The first fat-soluble active ingredient 50' is different from the second fat-soluble active ingredient 50", or the first fat-soluble active ingredient 50' is different in concentration from the second fat-soluble active ingredient 50".

The first fat-soluble active ingredient 50', is contained in the lipophilic phase, i.e. in the outer phase 22 of the individual molecules composing the water in oil cream 20'. The second fat-soluble active ingredient 50', is contained in the lipophilic phase, i.e. in the inner phase 31 of the individual molecules composing the oil in water cream 30".

In order to ensure the separation over time between creams 20', 30", avoiding mixing the fat-soluble active ingredients 50', 50", among the fat-soluble active ingredients there is at least one lipophobic separation layer.

In this example, among the fat-soluble active ingredients 50', 50" is only one lipophobic separation layers, represented by the outer phase 32 of the individual molecules that make up the second oil in water cream 30".

Figure 8 shows a sixth embodiment example of the packaging system according to the invention.

In this example, a layer of a first oil in water cream 30' containing a first water soluble active ingredient 40' is contacted with a layer of a second water in oil cream 20" containing a second fat-soluble active ingredient 50".

The first 40' and the second 50" active ingredient are different.

The first water soluble active ingredient 40', is contained in the hydrophilic phase, i.e. in the outer phase 32 of the individual molecules composing the oil in water cream 30'. The second fat-soluble active ingredient 50', is contained in the lipophilic phase, i.e. in the outer phase 22 of the individual molecules composing the water in oil cream 20'.

In order to ensure the separation over time between creams 30', 20", preventing the active ingredients from moving from one cream to the other, among the active ingredients there both a lipophobic separation layer and a hydrophobic separation layer.

In this example, between the active ingredients there is both a lipophobic separation layer, represented by the outer phase 32 of the individual molecules that make up the first oil in water cream 30', and a hydrophobic separation layer, represented by the outer phase 22 of the individual molecules that make up the second water in oil cream 20".

In summary therefore, the packaging system for dependency-free use of products for topical application according to the present invention provides the arrangement in layers, overlapped in the same container 10, of two or more creams 20, 30 different in formulation and/or concentration of active ingredients 40, 50, in which the layered creams are dispensed and used in succession to alternate the use thereof and to use them in progressively different doses and concentrations of active ingredients 40, 50.

Container 10 for example is a bottle, a metal or plastic tube, a flexible pouch.

Container 10 is provided with a single dispensing opening.

In an embodiment example, shown in figures 11 and 12, container 10 is airless, i.e. the type able to dose creams 20, 30 without using pumps with draft on the bottom. Container 10 is superiorly provided with a dispensing valve 15, devoid of draft, and inferiorly with an inner movable piston 16 which raises upwards, pushing the cream to allow the dispensing thereof, due to the vacuum created by the dispensing valve itself. This system avoids mixing creams 20, 30 even in case of shaking or tilting of container 10 as there will never be free space within container 10 to allow the physical movement of the creams.

Preferably, creams 20, 30 are for topical application and in the form of emulsions, ointments, pastes, salves, gels, lipogels and the like.

Preferably, creams 20, 30 can be used for dermatological, cosmetic, or pharmacological purposes.

In one example, creams 20, 30 are coloured differently to allow a visual identification of their nature, as regards the different appearance, fragrance, taste or aroma.

In one example, creams 20, 30 arranged in succession in the same container 10 and dispensed progressively contain doses and/or concentrations of active ingredients 40, 50 increasing from the first layer 20' to the last layer 30''''.

In one example, creams 20, 30 arranged in succession in the same container 10 and dispensed progressively contain doses and/or concentrations of active ingredients 40, 50 decreasing from the first layer to the last layer.

Creams 20, 30 are therefore layered in container 10 in such a way as to be dispensed consecutively to alternate the use thereof (in case of continuous applications) or use them in progressively stronger concentrations (in case of periodic applications) in order to avoid the phenomenon of dependency.

Taking as an example a cosmetic product used every day continuously (figure 7), in a single container 10 are arranged two different creams 20, 30, each containing a complex of active substances completely and qualitatively different from each other. In container 10, the two creams 20, 30 are arranged in layers. In this way, two different creams are dispensed in succession: a first cream 20, then when this is over, a second cream 30. This system ensures an alternate use of different products. In doing so, the skin is not able to get used to different active ingredients contained in the two different creams, hereby nullifying the dependency effect.

Such a packaging system and use can also be contemplated for dermatological and cosmetic products for periodic use (figure 8). For a progressive intensity treatment, in the same container 10 are arranged multiple layers 20', 30", 20''', 30'''' of the same product, containing an increasing dose or concentration of active ingredients from the first layer 20' to the last layer 20''. Therefore, with a single container 10 a product is dispensed progressively in doses and concentrations gradually stronger to obviate the dependency.

In summary, the packaging system according to the present invention comprises:
- a container 10 with a single dispensing opening 11,
- at least two creams 20, 30 with different formulations and/or concentrations of active ingredients 40, 50, arranged in superposed layers in container 10, in which the cream is of the oil in water 30 or water in oil 20 type;
- at least one active ingredient 40, 50 contained in the inner phase 21, 31 or in the outer phase 22, 32 of the individual molecules composing cream 20, 30 itself.

In particular, the packaging system is characterised in that between the creams 20, 30 there is at least one separation layer able to repel said active principle 40, 50 in such a way that the active ingredients 40, 50 contained in the creams 20, 30 do not spread, passing from one cream to the other and making altogether vain the subdivision in layers. Moreover, the separation layer is composed of the outer phase 22, 32 of the individual molecules that make up cream 20, 30 itself.

Innovatively, a packaging system according to the present invention allows arranging layers of different products (as formulation and/or dosage of active substances) in contact with each other and ensuring the separation thereof over time.

Advantageously, a packaging system according to the present invention prevents the active ingredients in the creams from spreading and moving from one cream to the other, making the division into layers altogether vain.

Advantageously, a packaging system according to the present invention allows preventing the spread of active substances from a product (cream or emulsion) to another through points or areas of mutual contact that are created during their layering within the container.

In conclusion, with a packaging system according to the present invention there will be three layering options:
- same type of creams:
   1. O/W with O/W, both with aqueous outer contact layer and oily inner layer: all that is aqueous and water soluble will move from one cream to the other by spreading; all that is oily and oil- soluble will not move from one cream to the other because there is a double aqueous separation layer;
   2. W/O with W/O, both with oily outer contact layer and aqueous inner layer: all that is oily and oil soluble will move from one cream to the other by spreading; all that is aqueous and water soluble will not move from one cream to the other because there is a double oily separation layer;
- different type of creams:
   3. O/W with A/W, an oily outer contact layer in contact with an aqueous outer contact layer: no passage of fat-soluble and/or water soluble substances occurs between one cream and the other because there is both an oily separation layer and an aqueous separation layer.

It is clear that a man skilled in the art may make changes to the packaging system according to the present invention in order to meet incidental needs, all falling within the scope of protection defined in the following claims.

## Claims

1. Packaging system for dependency-free use of products for topical application, comprising:
- a container (10) with a single dispensing opening (11),
- at least a first cream and a second cream with different formulations and/or concentrations of active ingredients (40,50), arranged in superposed layers in the container (10), in which the first cream is of the oil in water (30) type and the second cream is of the water in oil (20) type;
- at least one active principle (40,50) contained in the inner phase (31) or in the outer phase (32) of the individual molecules composing said first cream;
- at least one active principle (40,50) contained in the inner phase (21) or in the outer phase (22) of the individual molecules composing said second cream; **characterised in that** between the creams (20,30) there is at least one separation layer able to repel said active principle (40, 50),
wherein said separation layer is the outer phase (32) of the molecules composing the first cream (30), that is a lipophobic separation layer for the active principle (50) contained in the second cream (20), and/or the outer phase (22) of the molecules composing the second cream (20), that is a hydrophobic separation layer for the active principle (40) contained in the first cream (30), in such a way that the active ingredients (40,50) contained in the creams (20,30) do not spread, passing from one cream to the other and making altogether vain the subdivision in layers.

2. Packaging system according to claim 1, wherein the active principle is of the water-soluble type (40), contained in the hydrophilic phase of the individual molecules composing said creams, and the separation layer is of the hydrophobic type.

3. Packaging system according to claim 1, wherein the active principle is of the fat-soluble type (50), contained in the lipophilic phase of the individual molecules composing said creams, and the separation layer is of the lipophobic type.

4. Packaging system according to any of the preceding claims, wherein the creams (20, 30) both contain a water-soluble (40) or a fat-soluble active ingredient (50).

5. Packaging according any of the claims from 1 to 3, wherein the first cream (30) contains a water-soluble active principle (40) and the second cream (20) contains a fat-soluble active ingredient (50).

6. Packaging system according to any of the preceding claims, wherein at least one of the active principles (40, 50) is contained in the outer phase (22,32) of the individual molecules composing the creams (20, 30).

7. Packaging system according to claim 6, wherein the active ingredient is water-soluble (40') and contained in the outer phase (32) of the first cream (30), and the outer phase (22) of the second cream (20) is the hydrophobic separating layer.

8. Packaging system according to claim 6 or 7, wherein the active ingredient is fat-soluble (50) and contained in the outer phase (22) of the first cream (20), and the outer phase (32) of the second cream (30) is the lipophobic separating layer.

9. Packaging system according to any of the preceding claims, wherein the creams (20,30) arranged in succession are coloured differently to allow a visual identification of their nature, as regards the different appearance, fragrance, taste or aroma.

10. Packaging system according to any of the preceding claims, wherein the creams (20, 30) arranged in succession contain concentrations of active ingredients (40, 50) increasing or decreasing from the first layer (20') to the last layer (30'''').

## Patentansprüche

1. Verpackungssystem zur abhängigkeitsfreien Verwendung von Produkten zur topischen Anwendung, umfassend:
- einen Behälter (10) mit einer einzigen Abgabeöffnung (11),
- zumindest eine erste Creme und eine zweite Creme mit unterschiedlichen Formulierungen und/oder Konzentrationen von Wirkstoffen (40,50), die in übereinanderliegenden Schichten in dem Behälter (10) angeordnet sind, wobei die erste Creme vom ÖI-in-Wasser-Typ (30) ist und die zweite Creme vom Wasser-in-Öl-Typ (20) ist;
- zumindest einen aktiven Stoff (40,50), der in der inneren Phase (31) oder in der äußeren Phase (32) der einzelnen Moleküle enthalten ist, welche die erste Creme bilden;
- zumindest einen aktiven Stoff (40,50), der in der inneren Phase (21) oder in der äußeren Phase (22) der einzelnen Moleküle enthalten ist, welche die zweite Creme bilden;
**dadurch gekennzeichnet, dass** es zwischen den Cremes (20, 30) zumindest eine Trennschicht gibt, die in der Lage ist, den aktiven Stoff (40, 50) abzustoßen,
wobei die Trennschicht die äußere Phase (32) der Moleküle ist, welche die erste Creme (30) bilden, d.h. eine lipophobe Trennschicht für den in der zweiten Creme (20) enthaltenen aktiven Stoff (50), und/oder die äußere Phase (22) der Moleküle ist, welche die zweite Creme (20) bilden, d.h. eine hydrophobe Trennschicht für den in der ersten Creme (30) enthaltenen aktiven Stoff (40), so dass die Wirkstoffe (40, 50), die in den Cremes (20,30) enthalten sind, sich nicht verteilen, von einer Creme zur anderen übergehen und die Unterteilung in Schichten insgesamt nutzlos bzw. vergeblich machen.

2. Verpackungssystem nach Anspruch 1, wobei der aktive Stoff vom wasserlöslichen Typ (40) ist, der in der hydrophilen Phase der einzelnen Moleküle enthalten ist, welche die Cremes bilden, und die Trennschicht vom hydrophoben Typ ist.

3. Verpackungssystem nach Anspruch 1, wobei der aktive Stoff vom fettlöslichen Typ (50) ist, der in der lipophilen Phase der einzelnen Moleküle enthalten ist, welche die Cremes bilden, und die Trennschicht vom lipophoben Typ ist.

4. Verpackungssystem nach einem der vorhergehenden Ansprüche, wobei die Cremes (20, 30) beide einen wasserlöslichen (40) oder einen fettlöslichen Wirkstoff (50) enthalten.

5. Verpackung nach einem der Ansprüche 1 bis 3, wobei die erste Creme (30) einen wasserlöslichen aktiven Stoff (40) und die zweite Creme (20) einen fettlöslichen Wirkstoff (50) enthält.

6. Verpackungssystem nach einem der vorhergehenden Ansprüche, wobei zumindest einer der aktiven Stoffe (40, 50) in der äußeren Phase (22, 32) der einzelnen Moleküle enthalten ist, welche die Cremes (20, 30) bilden.

7. Verpackungssystem nach Anspruch 6, wobei der Wirkstoff wasserlöslich (40') ist und in der äußeren Phase (32) der ersten Creme (30) enthalten ist, und die äußere Phase (22) der zweiten Creme (20) die hydrophobe Trennschicht ist.

8. Verpackungssystem nach Anspruch 6 oder 7, wobei der Wirkstoff fettlöslich (50) ist und in der äußeren Phase (22) der ersten Creme (20) enthalten ist, und die äußere Phase (32) der zweiten Creme (30) die lipophobe Trennschicht ist.

9. Verpackungssystem nach einem der vorhergehenden Ansprüche, wobei die nacheinander angeordneten Cremes (20, 30) unterschiedlich gefärbt sind, um eine visuelle Identifizierung ihrer Art hinsichtlich des unterschiedlichen Aussehens, Dufts, Geschmacks oder Aromas zu ermöglichen.

10. Verpackungssystem nach einem der vorhergehenden Ansprüche, wobei die nacheinander angeordneten Cremes (20, 30) Konzentrationen von Wirkstoffen (40, 50) enthalten, die von der ersten Schicht (20') zu der letzten Schicht (30"") zunehmen oder abnehmen.

## Revendications

1. Système de conditionnement pour une utilisation sans dépendance de produits destinés à l'application topique, comprenant :
- un récipient (10) présentant une seule ouverture de distribution (11),
- au moins une première crème et une deuxième crème ayant différentes formulations et/ou concentrations d'ingrédients actifs (40, 50), disposées en couches superposées dans le récipient (10), dans lequel la première crème est de type huile dans l'eau (30) et la deuxième crème est de type eau dans l'huile (20) ;
- au moins un principe actif (40, 50) contenu dans la phase interne (31) ou dans la phase externe (32) des molécules individuelles composant ladite première crème ;
- au moins un principe actif (40, 50) contenu dans la phase interne (21) ou dans la phase externe (22) des molécules individuelles composant ladite deuxième crème ;
**caractérisé en ce qu'**entre les crèmes (20, 30), il y a au moins une couche de séparation capable de repousser ledit principe actif (40, 50),
dans lequel ladite couche de séparation correspond à la phase externe (32) des molécules composant la première crème (30), qui est une couche de séparation lipophobe pour le principe actif (50) contenu dans la deuxième crème (20), et/ou à la phase externe (22) des molécules composant la deuxième crème (20), qui est une couche de séparation hydrophobe pour le principe actif (40) contenu dans la première crème (30), de telle manière que les ingrédients actifs (40, 50) contenus dans les crèmes (20, 30) ne diffusent pas, passant d'une crème à l'autre et rendant tout à fait vaine la subdivision en couches.

2. Système de conditionnement selon la revendication 1, dans lequel le principe actif est du type soluble dans l'eau (40), contenu dans la phase hydrophile des molécules individuelles composant lesdites crèmes, et la couche de séparation est de type hydrophobe.

3. Système de conditionnement selon la revendication 1, dans lequel le principe actif est du type liposoluble (50), contenu dans la phase lipophile des molécules individuelles composant lesdites crèmes, et la couche de séparation est de type lipophobe.

4. Système de conditionnement selon l'une quelconque des revendications précédentes, dans lequel les crèmes (20, 30) contiennent toutes deux un ingrédient actif soluble dans l'eau (40) ou liposoluble (50).

5. Système de conditionnement selon l'une quelconque des revendications 1 à 3, dans lequel la première crème (30) contient un principe actif soluble dans l'eau (40) et la deuxième crème (20) contient un ingrédient actif liposoluble (50).

6. Système de conditionnement selon l'une quelconque des revendications précédentes, dans lequel au moins l'un des principes actifs (40, 50) est contenu dans la phase externe (22, 32) des molécules individuelles composant les crèmes (20, 30).

7. Système de conditionnement selon la revendication 6, dans lequel l'ingrédient actif est soluble dans l'eau (40') et est contenu dans la phase externe (32) de la première crème (30), et la phase externe (22) de la deuxième crème (20) correspond à la couche de séparation hydrophobe.

8. Système de conditionnement selon la revendication 6 ou 7, dans lequel l'ingrédient actif est liposoluble (50) et est contenu dans la phase externe (22) de la première crème (20), et la phase externe (32) de la deuxième crème (30) correspond à la couche de séparation lipophobe.

9. Système de conditionnement selon l'une quelconque des revendications précédentes, dans lequel les crèmes (20, 30) disposées successivement sont de couleurs différentes pour permettre une identification visuelle de leur nature, en ce qui concerne les différents aspects, parfums, goûts ou arômes.

10. Système de conditionnement selon l'une quelconque des revendications précédentes, dans lequel les crèmes (20, 30) disposées successivement contiennent des concentrations d'ingrédients actifs (40, 50) croissantes ou décroissantes de la première couche (20') à la dernière couche (30"").
